(19) Europäisches Patentamt
European Patent Office
Office européen des brevets

(11) **EP 2 535 708 A1**

(12) **EUROPEAN PATENT APPLICATION**

(43) Date of publication:
**19.12.2012 Bulletin 2012/51**

(51) Int Cl.:
**G01N 33/28** (2006.01)

(21) Application number: **11169829.6**

(22) Date of filing: **14.06.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Fiat Powertrain Technologies S.p.A.**
**10135 Torino (IT)**

(72) Inventors:
• **Tucci, Vittorio Antonio**
  **13135 Torino (IT)**

• **Sidoti, Cinzia**
  **13135 Torino (IT)**
• **Vennettilli, Nando**
  **13135 Torino (IT)**
• **Garofalo, Fabio**
  **13135 Torino (IT)**
• **Zaehner, Werner**
  **13135 Torino (IT)**

(74) Representative: **Notaro, Giancarlo**
**Buzzi, Notaro & Antonielli d'Oulx**
**Via Maria Vittoria 18**
**10123 Torino (IT)**

(54) **Method for detecting the quality of the engine oil in a diesel engine and corresponding detection system**

(57)     A method for detecting the quality of engine oil (13) in an internal combustion engine (10), that comprises measuring at least the viscosity and the temperature (T) of the engine oil (13).

According to the invention, such method comprises estimating (M2) an aging (A) of the engine oil (13) based on measured viscosity values ($\nu_m$),
evaluating (340) whether the estimated aging (A) of the engine oil is indicative of dilution of the engine oil,
if so applying (363) a dilution estimation model (M1) to calculate the dilution based on said measured viscosity values ($\nu_m$).

Fig. 5

**Description**

Field of the invention

The present invention refers to methods and systems for detecting the quality of engine oil in an internal combustion engine, which comprise measuring at least the viscosity and temperature of the motor oil.

General technical problem and description of the known art

Detecting oil quality in an internal combustion engine is important for carrying out an engine management strategy that guarantees safety and avoids failures.

Methods are generally known, for example from the US patent application US2009139484A1, that use an oil quality sensor to monitor deterioration, evaluating parameters comprising, for example oil viscosity, to provide an indicator of the quality. If such indicator has a value below a calibratable threshold, the user is prompted to change the oil.

Such assessment strategy does not represent a complete and robust solution with respect to the factors that can influence the evaluation of oil quality, for example influence the viscosity.

A known factor that influences such evaluation is the dilution of engine oil.

The dilution of engine oil with fuel is a phenomenon for which monitoring with a specific assessment function is envisioned to avoid mechanical damage (due to reduction of the lubricating capacity of the oil) or auto-combustion phenomena with consequent engine over-revving (due to the increase in the vapour pressure of the oil in the sump that causes ignition of oil drawn into the engine).

In automobile engines the source of dilution is the regeneration of the anti-particulate filter, caused by the use of very late injections that strike the chamber walls and seep into the sump.

In diesel engines, for example engines of the Cursor type developed and produced by Fiat Powertrain technologies, dilution may be caused by fuel leakage from the rail embedded in the head and then entering the oil bath. Diesel fuel for example seeps through play in the injection system from the loosening of fittings, tubes and other such elements.

To evaluate the dilution, it is known for example that monitoring the gas pressure in the crankcase, assuming that such pressure increases with an increase in the vapour pressure of the oil due to seepage of diesel fuel. Seepage superior to 30 1/h can be detected by a strategy that monitors rail pressurisation by refilling and emptying times of the diesel fuel circuit. Crankcase pressure is a late indicator of the dilution phenomenon that only allows detection of dilution at a high percentage, not allowing timely safety action that protects the engine from auto-combustion and seizing phenomena.

Object of the invention

The object of the present invention is that of overcoming the previously described problems with the known art. In particular, the object of the present invention is that of detecting dilution of the oil by fuel, taking into account the oil quality, in particular by means of a specific sensor.

Summary of the invention

The object of the present invention is achieved by a method for detecting engine oil quality having all of the characteristics listed at the beginning of the present description and also characterised in comprising estimation of the aging of the engine oil based on the measured viscosity values, evaluating whether the estimated aging of the engine oil is indicative of dilution of the engine oil; if so applying a dilution estimation model to calculate the dilution based on said measured viscosity values.

The invention also concerns a corresponding oil quality detection system.

Brief description of the drawings

The invention will now be described with reference to the annexed drawings, provided by way of nonlimiting example only, in which:

- figures 1, 2 and 3 are diagrams representative of the properties and quantities of engine oil used in the operation of the method according to the invention;
- figure 4 is a schematic diagram of a system implementing the method according to the invention;
- figure 5 is a flow diagram illustrating an embodiment of the method according to the invention.

Detailed description of the invention

Briefly, the method according to the invention envisions the use of information from an oil quality sensor to monitor the decrease in oil viscosity due to dilution by diesel fuel.

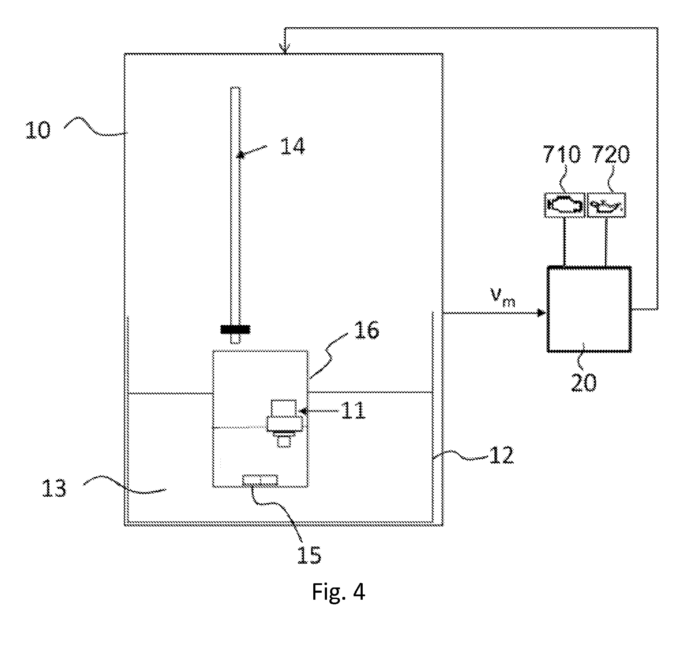

Figure 4 is a schematic diagram of a diesel engine 10, equipped with a sump 12 in which the engine oil 13 is found. In an oil filter 16 in said sump 12 an oil quality sensor 11 is located, that provides a measurement of viscosity of the engine oil 13 to an engine control unit 20. Such oil quality sensor 11 also provides a measurement of the dielectric constant of the oil, of its density and of its temperature. An example of this type of sensor, already present on the market, is the FPS2800 produced by Measurement Specialties.

Said engine control unit 20 controls the diesel engine 10 and is also adapt to emit alarms, for example turning on corresponding lights 710 and 720 and actuating procedures associated with them, as is better detailed below.

Above the oil sump a fuel rail 14 is shown. In the sump 12 an oil temperature sensor 15 for measuring an oil temperature T is also indicated. Such sensor is preferably comprised in the oil quality sensor 11.

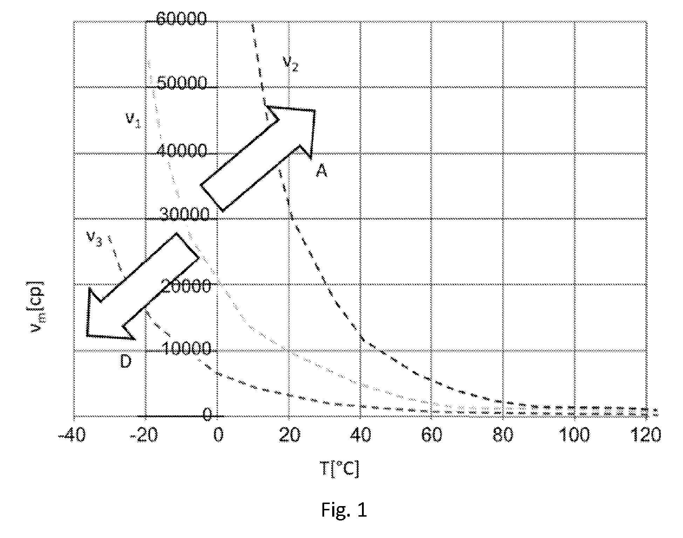

With reference to diagrams in figures 1, 2 and 3 trends and values of quantities relevant for the method according to the invention are presented.

Figure 1 shows a diagram representing the viscosity $v_m$ of the engine oil 13, measured by the oil quality sensor 11 as a function of the oil temperature T. That figure shows a first curve of viscosity $v_1$ as a function of temperature T measured under reference conditions, for example after an oil change with new oil, a second curve of viscosity $v_2$ as a function of temperature T with values greater than those in the first viscosity curve $v_1$, due to aging A of the engine oil 13, a third curve of viscosity $v_3$ as a function of temperature T with values less than the first viscosity curve $v_1$, due to the effect of dilution D of the engine oil 13. Hence, in general, for a given temperature T, the aging A of oil causes an increase in the viscosity measured $v_m$, while dilution D caused a decrease in the viscosity measured $v_m$.

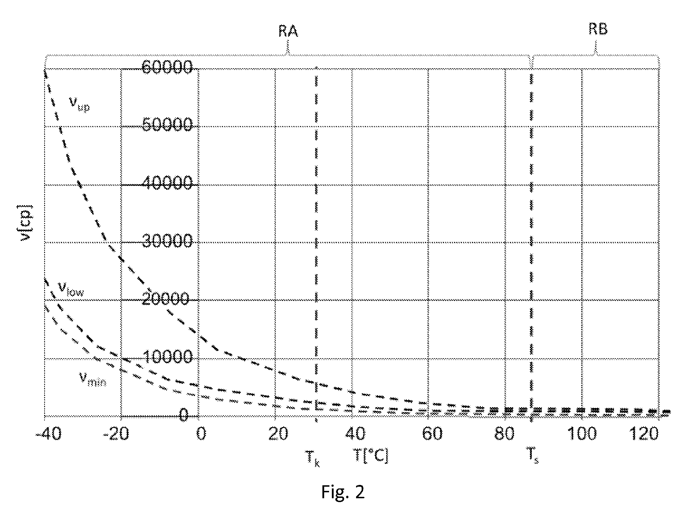

Figure 2 shows a diagram of a representative viscosity $v$ curve, regions and values used or relevant for the method according to the invention.

In particular in such diagram, $v_{low}$ indicates a lower limit curve and $v_{up}$ indicates an upper limit curve for the viscosity values. Such two curves delimit the area in which the various types of oil present on the market and potentially usable as engine lubricants can fall. The monitoring strategy aims to detect dilution by fuel of various types of oil, both if they are formulated for use at low temperature or formulated for use at high temperature. The viscosity curve $v_1$ mentioned in figure 1 for a given type of new oil will fall within the area delimited by the two curves.

A diagnostic viscosity threshold curve, below the lower limit curve $v_{low}$ is indicated with $v_{min}$. If values below such diagnostic threshold curve $v_{min}$ are measured, it is advantageous that the control unit 20 activate the on board diagnosis OBD light and a safety procedure that reduces engine performance, because there is a risk that the mechanical components for transmission of motion will seize.

In figure 2 a threshold temperature $T_s$ is indicated that divides the field of temperature associated with viscosity into two regions, a first region RA, and a second region RB. The threshold temperature $T_s$ represents the end of the engine heating phase and the consequent conditioning of the temperature of the cooling and lubricating liquids.

As can be seen, in the first region RA, the viscosity $v$ depends strongly on the temperature of the oil T. In said first region RA, measurement of the Viscosity gradient as a function of temperature has little relevance as indicator for dilution D, because the system has a naturally steep gradient. In such region the indicator of dilution is the absolute value of the viscosity.

In the second region RB instead, the viscosity gradient with respect to temperature T is near zero, i..e, the viscosity is substantially independent of the variations in temperature T. The value of the threshold temperature $T_s$ can vary according to conditions, but for example is greater than or equal to 85°C. According to an aspect of the invention, it is envisioned to operate in said second region RB to evaluate a gradient of viscosity $v$ as a function of time, exploiting the fact that the viscosity gradient $v$ as a function of temperature T has a very low or null value. A possible sudden variation of such gradient would be an indicator of a dilution of the oil caused by fuel seepage.

Also indicated in figure 2 is a key-on temperature $T_k$, i.e., the temperature of the engine switched off at insertion of the key with consequent powering on of the instrument panel and activation of the control unit, which corresponds to about 30°C, or in general to ambient temperature. Such key-on temperature $T_k$, as can be seen, falls in the first region RA of temperature dependency, for which it represent a monitoring point and acquisition of the absolute viscosity value at a given calibratable temperature, which in the automatic controls is known as offset learning.

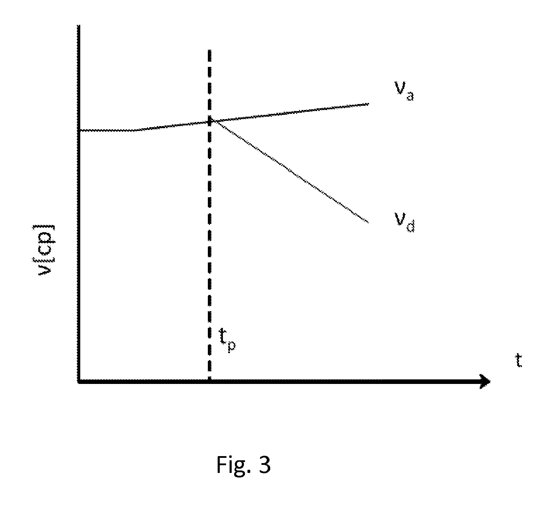

Figure 3 is also a diagram that shows the trend of the viscosity measured $v_m$ by the oil quality sensor 11 as a function of time t. Such time t can also be considered, as appropriate, equivalent to engine mileage. As can be seen, the measured viscosity $v_m$, initially substantially constant with time, then begins to increase from the effect of ageing A of the oil 13. A time after which the seepage of fuel into the oil can occur is indicated with tp, following for example, a maintenance service to the fuel injection system. A curve of viscosity as a function of aging in the absence of seepage after such time tp is indicated with $v_a$, while $v_d$ indicates a viscosity curve as a function of dilution caused by seepage that occurs after such time tp. As can be seen, viscosity increases with aging in the absence of dilution, while with dilution viscosity as a function of time decreases even if the aging process continues to take place, i.e., the dilution process is much faster and prevails over the aging process.

The method according to the invention envisions the use of measured viscosity $v_m$ from the oil quality sensor 11, inserting the measured values into two different viscosity models.

Use is envisioned of a dilution estimation model M1 that models the viscosity $v$ as a function of dilution D, of the oil temperature T and of the oil type OT, according to the exponential relationship:

$$v = e\{k1 - k2 * D + k3 * D * T + k4 + ...\}$$

where k1, k2, k3, k4 are empirically selected model constants. In particular the constant k4 assumes a different value according to the oil type OT.

By way of example, the estimation model of dilution M1 has the form:

$$v = e\left\{5.87 - 0.0396 * D + 0.000133 * D * T + \begin{vmatrix} 0.0899 & OT_1 \\ 0.1432 & OT_2 \\ -0.340 & OT_3 \\ 0.2909 & OT_4 \\ 0 & OT5 \end{vmatrix} + ...\right\}$$

where the various values of k4 are indicated according to the oil type $OT_1...OT_5$.

A second estimation model for aging M2 models the viscosity as a function of dilution D and of aging A, according the general relationship:

$$v = e\{k5 + k6 * A + k7 * D + ...\}$$

for example

$$v = e\{5.83 + 0.3897 * A + 0.036 * D + ...\}$$

Aging A can be expressed for example as a percentage of the oil change interval or of absolute time, expressed in hours.

Both the dilution estimation model and the aging estimation model M2 can have possible further parameters to take into consideration the influence of other physical parameters on viscosity.

The method for detecting engine oil quality, according to the invention, envisions in general measuring the viscosity values of engine oil 13 by means of an oil quality sensor 11 and inserting said measured viscosity values into the aging estimation model M2, obtaining an estimate or prediction of aging. In a system without seepage, at a given oil temperature the viscosity is increased and the aging estimation model M2 provides a positive value for aging A. If the viscosity should decrease, the model M2 would provide a negative aging value, i.e., a dilution. Therefore, having identified the inversion of the sign of the viscosity, i.e., a dilution process underway, the viscosity values provided by the oil quality sensor 11 are processed by the estimation model M1 of dilution D. Inverting the equation of the dilution estimation model M1 provides the level of dilution D of the oil as a percentage of the nominal volume of lubricant in the engine. Such values are compared with a maximum allowable dilution threshold, above which there is risk of auto-combustion of aspirated blow-by vapours, and the control unit activates the safety procedure, intended as limiting performance and suggesting the need for maintenance to the user.

If the temperature T of the engine oil 13 is higher than the temperature $T_s$, it is also envisioned that a viscosity gradient as a function of time be calculated, to instantaneously evaluate the effect of dilution on the oil quality.

Continuous monitoring of the absolute viscosity and dielectric constant is also envisioned. If the viscosity drops below a minimum calibratable threshold or if the dielectric constant exceeds a maximum calibratable threshold, the engine control unit actuates the safety procedure, intended as limiting performance and suggesting the need for maintenance to the user.

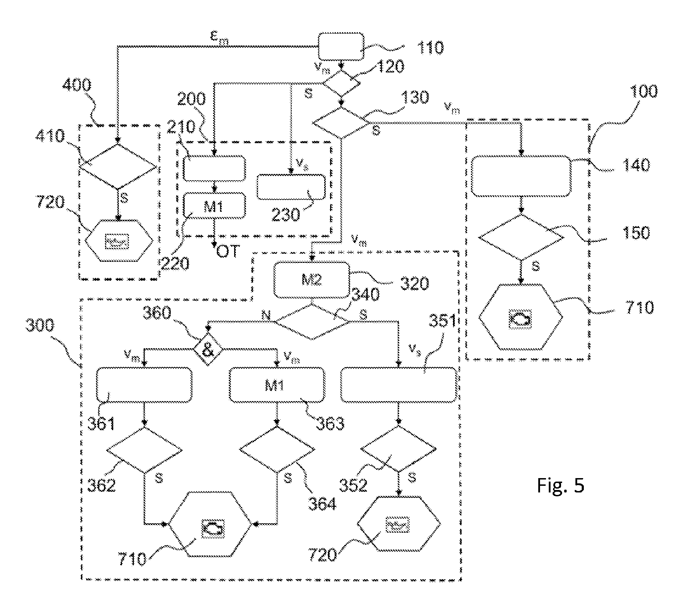

Figure 5 shows a flow diagram illustrating an embodiment of the method according to the invention.

Reference 110 indicates a measuring operation carried out by the quality sensor 11 that provides measured viscosity values $v_m$ and measured dielectric constant values $\varepsilon_m$.

The measured dielectric constant value $\varepsilon_m$ is submitted to a verification step 410 that performs a check of the absolute value of the dielectric constant $\varepsilon_m$. The dielectric constant increases with aging, for which safety actions are undertaken above a calibratable threshold. Thus, if the absolute value of the dielectric constant $\varepsilon_m$ is above a calibratable threshold dielectric constant value $\varepsilon_{th}$, in a step 720, an alarm is emitted, i.e., a change oil light is turned on.

Reference 200 indicates a preliminary procedure 200 for identifying the type of oil OT, which is carried out with a new or just serviced engine and therefore with new oil. Such procedure 200 may not be necessary when data on the oil type OT are supplied directly to the control unit. In such context if in a step 120, subsequent to the measuring step 110, the so-called key-on occurs, i.e., upon inserting the key with consequent powering on of the instrument panel and activation of the control unit, it is envisioned to verify whether this is the very first key-on (new vehicle) or the first key-on subsequent to an oil change, in order to evaluate if the oil is new. If so, the preliminary procedure 200 for identifying the type of oil OT is carried out only with a new or just serviced engine and therefore with new oil. Such procedure 200 may not be necessary when data on the oil type OT are supplied directly to the control unit.

Such procedure 200 envisions that the sensor 11 in step 110 supplies the measured viscosity $v_m$ measured by the oil quality sensor 11 and that in a step 210 the first dilution estimation model M1 is used with input data of said viscosity $v_m$ measured by the oil quality sensor 11. In this case the dilution D and aging A are null because the oil is new. Thus in the dilution estimation model M1 the only unknown is the constant k4 related to the oil type OT. Thus from step 210 a value is obtained corresponding to one of the above-mentioned constants corresponding to the oil type $OT_1$... $OT_n$, the values of which are stored for example in the control unit 20 of the vehicle. In step 220 identification of the oil type OT is completed.

Moreover, in the context of procedure 200 with new oil, in parallel with identification of the oil type OT, in step 230 the viscosity values $v_m$ measured by the oil quality sensor 11 are acquired to construct and store a baseline viscosity curve $v_s(T)$ as a function of temperature T of the new oil. This construction envisions acquiring viscosity values as the temperature T rises. Such curve is stored autoadaptively in the non-volatile memory of the engine control unit 20. The storage operation in the control unit 20, if not performed at key-on, is preferably performed at least at key-off, that is, when the instrument panel is turned off during the time between such operation and interruption of the supply to the engine control unit. Such time period is usually called the powerlatch time.

Successive to step 120, control is passed to an operation 130 that checks if the temperature T of the engine oil is higher than the threshold temperature $T_s$.

If such step 130 determines that the temperature T is lower than the threshold temperature $T_s$, for which the measured viscosity is subject to large fluctuations for small variations in the temperature T, an initial estimation procedure 300 is performed, which is usually started during the key-on phase, with the control unit supplied and the engine off and at the key-on temperature $T_k$ that, as was said, is for example about 30°C (ambient temperature). It is envisioned that the measured viscosity $v_m$ be supplied to the aging estimation model M2 in a step 320, which is performed continuously in the background having as input such measured viscosity $v_m$ and predicts the aging A.

If a negative aging value A is found in a verification step 340, corresponding to a decrease in viscosity, i.e., a dilution with respect to what was calculated based on the previously-stored values of the curve $v_s(T)$, then in a step 360 one or more of the following procedures is selected:

- a dilution calculation step 363 that envisions performing the dilution estimation model M1, estimating the dilution D as a function of the measured viscosity value $v_m$. In a subsequent step 364 it is determined if the dilution value D is above a calibratable threshold $D_{max}$, if so, a safety procedure 710 is activated, in particular the OBD light is activated with possible limitation of performance, for example engine torque.
- a quality monitoring step 361 that envisions in a successive step 362 determining if the measured viscosity values $v_m$ are below the values of the diagnostic threshold curve $v_{min}$. If so the safety procedure 710 is activated.

Such safety procedure 710 is preferably activated in case one of the two conditions 362 or 364 is found.

If at step 340 a positive aging value A is found, and therefore no dilution process but only normal use processes are occurring, the viscosity curve $v_s$ stored in the control unit 20 is updated, initialised to step 230, and the aging A is evaluated to determine if it is greater than an aging threshold value $A_{max}$. If so, an alarm 720 is emitted, i.e., the change oil light is turned on.

If in the verification step 130 it is determined that the temperature T of the oil 13 is higher than the threshold temperature $T_s$ and therefore in the second temperature region RB in which viscosity is substantially independent of said temperature T of the engine oil 13, performance of a gradient calculation procedure 100 is envisioned, which envisions a step 140 of calculating the gradient of the viscosity as a function of time, to evaluate if the value of such gradient is less than zero, thereby indicating the presence of dilution, in particular of seepage. In a step 150 it is also envisioned to determine if the calculated viscosity gradient, i.e., the partial derivative or incremental ratio of the viscosity with respect to time t, are greater than a threshold value. If so the safety procedure 710 is activated.

In the context of the procedure 100 the estimation models M1 and M2 continue to be carried out with the same logic described previously, for example the procedure 300 is performed by the engine control unit 20, even if for an oil temperature T almost constant in that the engine is at running temperature, or in any case the aging values A are calculated using the aging estimation model M2 and dilution values D using the dilution estimation model M1 at such almost constant oil temperature T with the engine at running temperature.

In general, the method envisions estimation of the aging A of the engine oil 13 based on measured viscosity values $v_m$, evaluating if the estimated aging A of the engine oil is indicative of dilution of the engine oil, if so applying the dilution estimation model M1 to calculate the dilution on the basis of said measured viscosity values $v_m$. The occurrence of the required conditions in steps 120 and 130 to perform the preliminary procedure 200 and calculate a gradient 100 provide additional estimates of engine oil parameters possibly susceptible of actuating alarms or safety measures; the procedure 400 also provides a parallel evaluation based on the dielectric constant that is susceptible of actuating alarms or safety measures.

Consequently, if at step 130 it is verified that the temperature is in the region RA of temperature dependence, a preliminary evaluation is performed, verifying whether the values of the measured viscosity curve are above the values of the viscosity curve measured in the preceding cycle (step 340). If so, the stored curve is simply updated and used as the new baseline in the successive operation cycle. The updating process continues until the maximum admissible use is reached. Step 352 performs such assessment, possibly suggesting an oil change. If negative, i.e., in case of dilution, a dilution estimate using a mathematic model and monitoring of the absolute value of viscosity, which allows verification that the viscosity and estimated dilution in such region do not exceed danger thresholds.

If in step 130 it is verified that the temperature is in region RB, where there is no substantial variation of the oil temperature, the operation of estimating the aging of the engine oil based on measured viscosity values comprises an estimation of the variation in viscosity as a function of time, which allows instantaneous identification of the presence of seepage, applying the dilution estimation model M1 to estimate the dilution with precision.

Thus, the advantages of the proposed solution are clear from the preceding description.

The method according to the invention, by monitoring the viscosity gradient and calculating the percentage of dilution through a model from the information on absolute viscosity allows detection of oil quality and dilution of the oil by fuel with use of only one oil quality sensor, which monitors possible worsening of the lubricating property of the oil. This allows coverage of dilution phenomena for a wide range of oils and fuels of different grades. The model allows the percentage dilution to be calculated for every type of oil.

Moreover, this method advantageously allows rapid detection of seepage of diesel fuel by means of a viscosity gradient that identifies it through an instantaneous change in the slope of the viscosity line. This also allows detection of small quantities of fuel. In general such rapid detection allows rapid adoption of appropriate recovery or safety actions, aimed at protecting the engine.

The monitoring of oil aging can be advantageously performed through the dielectric constant provided by the sensor. If the dielectric constant is above a calibratable threshold, the engine control unit suggest that the oil be changed.

The proposed method advantageously envisions estimation of dilution covering the entire range of oil temperatures from -40°C to 120°C.

With respect to solutions that follow only the absolute viscosity values below a certain threshold, the proposed method is more complete and robust, allowing such measurements with gradient and with dilution model so to cover two of the principal possible engine failures, i.e., the generation of blow-by due to the increase in oil level in the sump, and seizing due to loss of lubrication capacity of the oil. Blow-by could not be detected with a single measurement of the absolute value of the viscosity, while seizing would only be detectable for a prescribed and experimentally tested range of oils.

The method is particularly advantageous in particular engine layout configurations, for example engines with a common rail installed inside the head.

The method according to the invention allows in general limiting the costs of maintenance services, of mechanical failures caused by reduced lubricating capacity of the oil, of auto-combustion due to aspiration of oil from the blow-by circuit.

Naturally, the details of implementation and the embodiments may vary widely with respect to what is described and illustrated without thereby departing from the field of protection of the present invention, as defined in the annexed claims.

The proposed method extends also to engines on which dilution of oil is due to regeneration of the anti-particulate filter through very late injections that strike the walls of the combustion chamber removing the lubricating film and seeping into the sump.

The operations of the proposed method are preferably performed by processor means, in particular a microprocessor of the control unit, that also acquires the signal from the sensor. Naturally, it is possible that such processing be performed by other processor means available in the vehicle.

Evaluation of the gradient above the threshold temperature is instantaneous, i.e., such evaluations can be envisioned continuous according to the frequency with which quality information is sent by the oil quality sensor.

## Claims

1. A method for detecting the quality of engine oil (13) in an internal combustion engine (10), that comprises measuring at least the viscosity and the temperature (T) of the engine oil (13),
**characterised in that**
it comprises estimating (M2) an aging (A) of the engine oil (13) based on measured viscosity values $v_m$),
evaluating (340) whether the estimated aging (A) of the engine oil is indicative of dilution of the engine oil,
if so applying (363) a dilution estimation model (M1) to calculate the dilution based on said measured viscosity values ($v_m$).

2. The method according to claim 1, **characterised in that** it envisions measuring the temperature (T) of the engine oil (13) and comparing it (130) to a threshold temperature ($T_s$) at which the viscosity is substantially independent from said temperature (T) of the engine oil (13).

3. The method according to claim 2, **characterised in that** if said temperature (T) of the engine oil is higher than said threshold temperature ($T_s$) said operation of estimating (M2) the aging (A) of the engine oil (13) based on measured viscosity values ($v_m$) comprises calculating a viscosity gradient as a function of time as a function of said measured viscosity values ($v_m$).

4. The method according to claim 3, **characterised in that** if the value of said Viscosity gradient as a function of time surpasses (150) a given threshold value a safety procedure is activated (710), in particular a limitation of engine performance.

5. The method according to claim 3 or 4, **characterised in that** it comprises continuously calculating (320) said gradient of viscosity as a function of time as a function of said measured viscosity values ($v_m$).

6. The method according to claim 2, **characterised in that** when said operation of evaluating (340) whether the estimated aging (A) of the engine oil is indicative of dilution of the engine oil, it envisions, if the temperature (T) of the engine oil is lower that said threshold temperature, comparing the aging corresponding to stored viscosity values ($v_s$) with the aging corresponding to measured viscosity values ($v_m$).

7. The method according to claim 6, **characterised in that**, when said operation of evaluating (340) is indicative of dilution of the engine oil (13), it comprises applying (363) a dilution estimation model (M1) to calculate the dilution based on said measured viscosity values ($v_m$) and actuating an alarm signal or a safety procedure (720) if the calculated dilution (D) of the engine oil is greater than a given threshold ($D_{max}$).

8. The method according to claim 6 or 7, **characterised in that**, if such evaluation operation (340) is indicative of dilution of the engine oil (13), it comprises verifying if said measured viscosity values ($v_m$) are below the values of a diagnostic threshold curve ($v_{min}$) and, if not actuating an alarm signal (720) or a safety procedure (710).

9. The method according to claim 6, **characterised in that**, if said evaluation operation (340) is not indicative of a dilution of the engine oil (13), it comprises updating (351) the stored viscosity curve values ($v_s$) with said measured values ($v_m$) and verifying if the aging (A) is greater that a maximum value ($A_{max}$), if so actuating an alarm signal (720).

10. The method according to one of the preceding claims, **characterised in that** it envisions verifying (120) if the oil is new and performing said dilution estimation model (M1) with null dilution to calculate the type of oil (OT).

11. The method according to one of the previous claims, **characterised in that** it comprises measuring (110) a dielectric constant ($\varepsilon_m$) of the oil and verifying (410) if said dielectric constant ($\varepsilon_m$) is greater than a calibratable dielectric constant threshold value ($\varepsilon_{th}$), if so actuating an alarm signal (720).

12. The method according the one of the previous claims, **characterised in that** said engine (10) is a diesel engine.

**13.** The method according to one of the previous claims, **characterised in that** said dilution estimation model (M1) models the viscosity (v) according to an exponential function of the dilution (D), of the temperature (T) and of the type of oil (OT) and said aging estimation model (M2) models the viscosity (v) as a function of the dilution (D) and of the aging (A).

**14.** A system for detecting the quality of engine oil (13) in an internal combustion engine (10), comprising one or more sensors adapted for measuring the viscosity of engine oil (13) and the temperature (T) of engine oil, processor means in particular in the engine control unit for acquiring the signals from said sensors (11) and evaluating said quality **characterised in that** said processor means (15) are configured for performing the method according to one or more of the claims from 1 to 13.

**15.** The system according to claim 14, **characterised in that** said processor means (15) are comprised in an engine control unit (20) comprising a non-volatile memory.

Fig. 1

Fig. 2

Fig. 3

Fig. 4

Fig. 5

**EUROPEAN SEARCH REPORT**

Application Number

EP 11 16 9829

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 10 2006 059071 A1 (BOSCH GMBH ROBERT [DE]) 19 June 2008 (2008-06-19) * the whole document * | 1-15 | INV. G01N33/28 |
| X | DE 10 2006 038968 A1 (SIEMENS AG [DE]) 28 February 2008 (2008-02-28) | 14 | |
| A | * paragraph [0024] - paragraph [0032] * | 1 | |
| A | EP 1 742 050 A2 (DELPHI TECH INC [US]) 10 January 2007 (2007-01-10) * paragraph [0017] - paragraph [0026] * | 1,14 | |
| A | US 5 750 887 A (SCHRICKER DAVID R [US]) 12 May 1998 (1998-05-12) * column 5, line 19 - line 37; figure 9 * | 1,14 | |
| A | US 5 604 441 A (FREESE V CHARLES E [US] ET AL) 18 February 1997 (1997-02-18) * column 29, line 51 - column 30, line 34 * | 1,14 | |
| A | DE 100 10 976 A1 (BOSCH GMBH ROBERT [DE]) 13 September 2001 (2001-09-13) * column 2, line 68 - column 3, line 16; figure 2 * | 1,14 | **TECHNICAL FIELDS SEARCHED (IPC)** G01N |

The present search report has been drawn up for all claims

| Place of search | Date of completion of the search | Examiner |
|---|---|---|
| Munich | 11 November 2011 | Purdie, Douglas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 11 16 9829

This annex lists the patent family members relating to the patent documents cited in the above-mentioned European search report.
The members are as contained in the European Patent Office EDP file on
The European Patent Office is in no way liable for these particulars which are merely given for the purpose of information.

11-11-2011

| Patent document cited in search report | | Publication date | Patent family member(s) | | Publication date |
|---|---|---|---|---|---|
| DE 102006059071 A1 | | 19-06-2008 | DE 102006059071 A1<br>WO 2008071639 A1 | | 19-06-2008<br>19-06-2008 |
| DE 102006038968 A1 | | 28-02-2008 | DE 102006038968 A1<br>EP 2057462 A1<br>JP 2010501768 A<br>KR 20090041443 A<br>US 2010307230 A1<br>WO 2008022987 A1 | | 28-02-2008<br>13-05-2009<br>21-01-2010<br>28-04-2009<br>09-12-2010<br>28-02-2008 |
| EP 1742050 A2 | | 10-01-2007 | EP 1742050 A2<br>US 2007006642 A1 | | 10-01-2007<br>11-01-2007 |
| US 5750887 A | | 12-05-1998 | AU 5194198 A<br>DE 19781517 T1<br>JP 2000506984 A<br>US 5750887 A<br>WO 9822818 A1 | | 10-06-1998<br>18-03-1999<br>06-06-2000<br>12-05-1998<br>28-05-1998 |
| US 5604441 A | | 18-02-1997 | AU 711133 B2<br>AU 5184796 A<br>CA 2215164 A1<br>EP 0811169 A1<br>JP 3804976 B2<br>JP H11502304 A<br>JP 2006177974 A<br>JP 2006183670 A<br>JP 2006194905 A<br>US 5604441 A<br>WO 9628742 A1 | | 07-10-1999<br>02-10-1996<br>19-09-1996<br>10-12-1997<br>02-08-2006<br>23-02-1999<br>06-07-2006<br>13-07-2006<br>27-07-2006<br>18-02-1997<br>19-09-1996 |
| DE 10010976 A1 | | 13-09-2001 | DE 10010976 A1<br>EP 1266217 A1<br>JP 4700251 B2<br>JP 2003526784 A<br>US 2003172722 A1<br>WO 0167096 A1 | | 13-09-2001<br>18-12-2002<br>15-06-2011<br>09-09-2003<br>18-09-2003<br>13-09-2001 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009139484 A1